(19) Europäisches Patentamt
European Patent Office
Office européen des brevets

(11) Numéro de publication: **0 094 102**
**B1**

# (12) FASCICULE DE BREVET EUROPEEN

(45) Date de publication du fascicule du brevet: **27.12.85**

(51) Int. Cl.⁴: **C 07 D 207/08**

(21) Numéro de dépôt: **83105420.0**

(22) Date de dépôt: **01.07.80**

(60) Numéro de publication de la demande initiale en application de l'article 76 CBE: **0024960**

## (54) Nouveaux dérivés de 1-(1-cyclohexénylméthyl) pyrrolidine et leur procédé de préparation.

(30) Priorité: **06.07.79 FR 7917610**
**01.04.80 FR 8007352**

(43) Date de publication de la demande:
**16.11.83 Bulletin 83/46**

(45) Mention de la délivrance du brevet:
**27.12.85 Bulletin 85/52**

(84) Etats contractants désignés:
**AT BE CH DE FR GB IT LI LU NL SE**

(56) Documents cités:
**US - A - 3 117 976**

(73) Titulaire: **SOCIETE D'ETUDES SCIENTIFIQUES ET INDUSTRIELLES DE L'ILE DE FRANCE, 46, Boulevard de Latour-Maubourg, F-75340 Paris Cedex 07 (FR)**

(72) Inventeur: **Thominet, Michel, 82 rue Bonaparte, F-75006 Paris (FR)**
Inventeur: **Franceschini, Jacqueline, 28 avenue Larroumes, F-94240 L'Hay-les-Roses (FR)**

(74) Mandataire: **Gallochat, Alain, SOCIETE D'ETUDES SCIENTIFIQUES ET INDUSTRIELLES DE L'ILE DE FRANCE 46, Boulevard de Latour Maubourg, F-75340 Paris Cedex 07 (FR)**

EP 0 094 102 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux dérivés de 1-(1-cyclohexènylméthyl) pyrrolidine de formule:

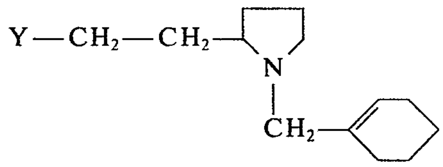

(I)

où Y représente un groupe hydroxy ou un atome de chlore.

Ces composés sont utiles comme intermédiaires de synthèse de phénoxyamines hétérocycliques substituées, de formule:

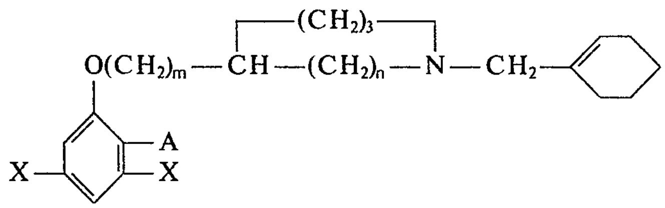

où:

A représente un atome d'hydrogène, un groupe alcoxy comportant 1 à 4 atomes de carbone ou alcényloxy comportant 2 à 6 atomes de carbone.

X représente un atome d'halogène.

m = 0 ou 2 et n = 0 ou 2
avec la condition que m + n = 2,

qui font l'objet de la demande de brevet EP-80 400 999-1 publiée sous le n° 24960.

L'invention concerne également un procédé de préparation des composés de formule (I), qui consiste à traiter la 2-hydroxyéthyl pyrrolidine par le 1-bromométhylcyclohexène puis à traiter la 1-(1-cyclohexènylméthyl) 2-hydroxyéthyl pyrrolidine ainsi formée, par le chlorure de thionyle pour obtenir la 1-(1-cyclohexènylméthyl)2-chloroéthyl pyrrolidine.

La présente invention est illustrée par les exemples suivants:

## Exemple I

### 1-(1-cyclohexènylméthyl)2-hydroxy éthyl pyrrolidine

Dans un ballon de 500 ml muni d'un agitateur, d'un thermomètre, d'un réfrigérant et d'une ampoule à brome on introduit: 16,4 g de 2-hydroxyéthyl pyrrolidine (0,143 mole), 84,3 ml de potasse alcoolique 1,78 N (0,150 mole) et goutte à goutte, 28 g de 1-bromométhyl cyclohexène (pureté ~ 90%) (0,160 mole). La température passe de 20° à 55°C et il se forme un précipité. On laisse réagir une heure puis filtre les sels et évapore à sec le filtrat. L'huile résiduelle est reprise dans 150 ml d'eau. On acidifie à l'acide chlorhydrique jusqu'à pH = 1 et extrait avec deux fois 100 ml d'ether éthylique.

La phase aqueuse est alcalinisée à la soude puis extraite avec trois fois 100 ml d'éther. Ces extraits sont séchés sur sulfate de magnésie, filtrés puis évaporés à sec.

L'huile résiduelle est distillée sous vide: P. E. 2 mmHg (266 Pa): 100–107°C
$n_D^{20}$ = 1,506 – Rendement: 20,7 g.

## Exemple II

### Chlorhydrate de 1-(1-cyclohexènylméthyl)2-chloro éthyl pyrrolidine

Dans un ballon de 250 ml muni d'un agitateur, d'un thermomètre, d'un réfrigérant et d'une ampoule à brome, on introduit 17,8 g de 1-(1-cyclohexènyl méthyl)2-hydroxy éthyl pyrrolidine (0,085 mole), 50 ml de chloroforme et, goutte à goutte, en maintenant la température vers 20–25°C par refroidissement, 15,3 ml (25 g) de chlorure de thionyle. On chauffe ensuite à reflux pendant trois heures.

On évapore la solution à sec puis reprend le résidu dans 20 ml de toluène et évapore à nouveau à sec sous vide.

Le solide résiduel est mis en suspension dans 100 ml d'acétate d'éthyle.

Les cristaux sont filtrés et séchés en étuve à 50°C.

On obtient 19,4 g (87 %) de produit fondant à 122°C.

**Revendications pour les Etats contractants: BE, CH, DE, FR, GB, IT, LI, LU, NL, SE**

1. Dérivés de 1-(1-cyclohexènylméthyl) pyrrolidine de formule:

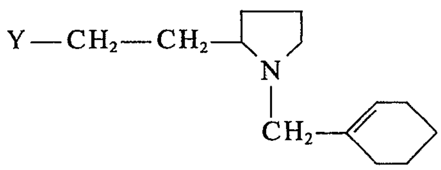

(I)

dans laquelle Y représente un groupe hydroxy ou un atome de chlore.

2. Procédé de préparation des composés de formule (I), selon la revendication 1, qui consiste à traiter la 2-hydroxyéthyl pyrrolidine par le 1-bromométhyl cyclohexène puis à traiter la 1-cyclohexènylméthyl 2-hydroxyéthyl pyrrolidine obtenue par le chlorure de thionyle.

**Revendication pour l'Etat contractant: AT**

Procédé de préparation de dérivés de 1-(1-cyclohexénylméthyl) pyrrolidine de formule:

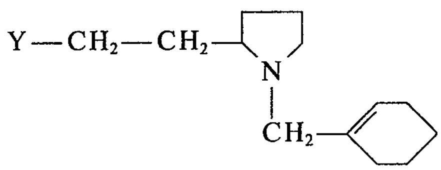

dans laquelle Y représente un groupe hydroxy ou un atome de chlore, qui consiste à traiter la 2-hydroxyéthyl pyrrolidine par le 1-bromométhylcyclohexène puis à traiter la 1-(1-cyclohexènylméthyl)-2-hydroxyéthyl pyrrolidine obtenue par le chlorure de thionyle.

**Patentansprüche für die Vertragsstaaten: BE, CH, DE, FR, GB, IT, LI, LU, NL, SE**

1. Derivate des 1-(1-Cyclohexenylmethyl)-pyrrolidins der Formel

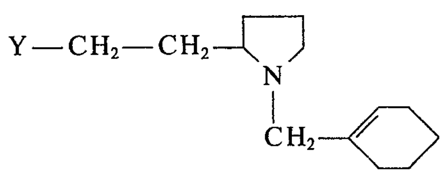

(I)

in der Y eine Hydroxygruppe oder ein Chloratom bedeutet.

2. Verfahren zur Herstellung von Verbindungen der Formel I nach Anspruch 1, dadurch gekennzeichnet, daß man 2-Hydroxyethylpyrrolidin mit 1-Brommethylcyclohexen umsetzt und anschließend das erhaltene 1-Cyclohexenylmethyl-2-hydroxyethylpyrrolidin mit Thionylchlorid behandelt.

**Patentanspruch für den Vertragsstaat: AT**

Verfahren zur Herstellung von Derivaten des 1-(1-Cyclohexenylmethyl)-pyrrolidins der Formel

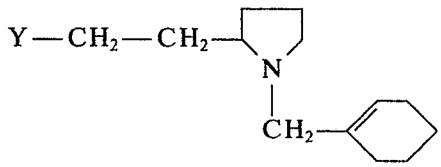

(I)

in der Y eine Hydroxygruppe oder ein Chloratom bedeutet, dadurch gekennzeichnet, daß man 2-Hydroxyethylpyrrolidin mit 1-Brommethylcyclohexen umsetzt und anschließend das erhaltene 1-(1-Cyclohexenylmethyl)-2-hydroxyethylpyrrolidin mit Thionylchlorid behandelt.

**Claims for the contracting States: BE, CH, DE, FR, GB, IT, LI, LU, NL, SE**

1. Derivatives of 1-(1-cyclohexenylmethyl)pyrrolidine of the following formula:

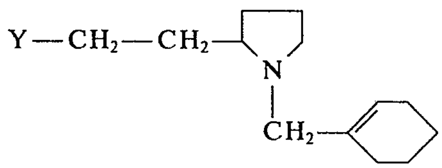

(I)

wherein Y represents a hydroxy group or a chlorine atom.

2. Process for the preparation of compounds of formula (I) according to claim 1 which comprises treating 1-hydroxyethyl pyrrolidine with 1-bromomethylcyclohexene and then treating the resulting 1-cyclohexenylmethyl 2-hydroxyethyl pyrrolidine with thionyl chloride.

**Claim for the contracting State: AT**

A process for the preparation of derivatives of 1-(1-cyclohexenylmethyl)pyrrolidine having the following formula:

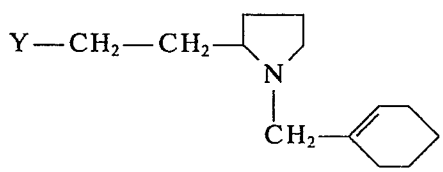

wherein Y represents a hydroxy group or a chlorine atom, which comprises treating 2-hydroxyethyl pyrrolidine with 1-bromomethyl cyclohexene and then treating the resulting 1-(1-cyclohexenylmethyl)-2-hydroxyethyl pyrrolidine with thionyl chloride.